Europäisches Patentamt

European Patent Office

Office européen des brevets

(11) Publication number: **0 017 853**
**B2**

(12) **NEW EUROPEAN PATENT SPECIFICATION**

(45) Date of publication of the new patent specification: 04.04.90

(51) Int. Cl.⁵: **C 12 N 1/16,** C 12 N 1/28, C 12 N 1/32

(21) Application number: 80101752.6

(22) Date of filing: 02.04.80

(54) A process for producing single cell protein material and culture.

(30) Priority: 12.04.79 US 29418
15.01.80 US 110457

(43) Date of publication of application:
29.10.80 Bulletin 80/22

(45) Publication of the grant of the patent:
24.10.84 Bulletin 84/43

(45) Mention of the opposition decision:
04.04.90 Bulletin 90/14

(84) Designated Contracting States:
BE DE FR IT NL SE

(56) References cited:
CA-A- 946 769
FR-A-2 400 061
GB-A-1 274 855
GB-A-1 348 304
GB-A-1 375 189
US-A-3 844 893

CHEMICAL ABSTRACTS, vol. 91, no. 7, 13th
August 1979, page 521, abstract no. 54608b,
Columbus, Ohio, U.S.A.

(73) Proprietor: PHILLIPS PETROLEUM COMPANY
5th and Keeler
Bartlesville Oklahoma 74004 (US)

(72) Inventor: Wegner, Eugene Herman
1509 Harris Drive
Bartlesville Oklahoma (US)

(74) Representative: Dost, Wolfgang, Dr.rer.nat.,
Dipl.-Chem. et al
Patent- und Rechtsanwälte Bardehle-
Pagenberg-Dost-Altenburg & Partner Postfach
86 06 20
D-8000 München 86 (DE)

(56) References cited:
CHEMICAL ABSTRACTS, vol. 90, no. 7, 12th
February 1979, page 468, no. 53149v, Columbus,
Ohio, U.S.A.

M. Kuraishi et al., Dechema Monographien Nr.
1704-1723, Microbiology applied to
Biotechnologie, Bd. 83, S. 111-123, 1979

M. Kuraishi et al., 2nd Intern. Symposium on
Microbial Growth on C1-Compounds,
September 12-16, 1977

Pushechino, UDSSR, "A Study on the
performance of Air-lift Fermenter in the
Production of methanol SCP"

EP 0 017 853 B2

# EP 0 017 853 B2

56 References cited:

H. Mori et al., J. Chem. Eng. Japan, 12, 313, 1979, "High density" cultivation of Biomass in fed-batch system with DO-STAT"

Ture and Appl. Chem., Vol. 51, p. 2259, Die Hegen, Bd. 2, 1962, S. 526

IUPAC-Guidelines for Testing of Single Cell Protein, destined as Protein Source for Animal Feed, Pure and Applied Chem., Vol. 51, p. 2259

"DIE HEFEN", BD. 2, TECHNOLOGIE DER HEFEN, 1962, P. 526

**Description**

Field of the Invention

The invention relates to a continuous process of producing single cell protein material and to novel yeast strains.

Background of the Invention

Efforts to relieve world-wide shortages of protein have included various bio-synthesis processes in which single cell protein (SCP) is obtained by the growth of one or another of a variety of microorganisms on various carbon-containing substrates.

The carbon energy substrates should be readily available, relatively cheap, uniform, and safe. Petroleum hydrocarbons have been employed as carbon energy source but have faced practical difficulties in lack of water-solubility and in the high consumption of molecular oxygen needed to assist in the microbial conversion. Other processes have centered on the use of oxygenated hydrocarbon derivatives as feedstocks due to their relative water-solubility and hence ease of handling in an aqueous ferment and in the substantially reduced molecular oxygen requirements for the microbial conversion-growth process.

However, a limiting factor in commercialization of single cell protein processes has been the necessity to control the ferment at relatively moderate cell densities, with but moderate yields of dried cells based on substrate consumed, and the consequent necessity to handle large amounts of total fermentation effluent liquor in order to recover the moderate amounts of SCP material. Handling large quantities of aqueous fermentation effluent liquor complicates concentration of the single cell protein product in such as centrifuges, as well as washing and drying steps.

Some processes in the past have concentrated on the culturing of bacterial because of the slightly higher crude protein contents of the cell as compared to the content obtainable from yeast in general. However, yeasts are widely available and relatively simply cultured. Yeast cells generally are slightly larger as compared to bacteria cells, and, hence, yeast cells tend to be more easily separated from the fermentation effluent liquor.

Discovery of means and methods to increase cell yields, and particularly to operate at high cell densities, would be highly desirable. The resultant handling of substantially less fermentation liquor effluent volume, for example, would mean large savings in reduced sizes of piping and pumps, reduced makeup water requirements with reduced sterilization requirements, and reduced requirements of equipment sizing and handling for coagulation and separation processes.

Summary of the Invention

It has been discovered a way to operate a continuous aerobic fermentation process employing yeast cultures so as to enable operation of the ferment in the fermentator at very high cell densities ordinarily unobtainable, and to produce high yields of yeast single cell protein product, by adding media containing high mineral salts concentrations to the ferment in the fermentor. Cell density is defined as the concentration of cells by weight on a dry basis per volume of ferment. The ferment is defined as the total volume of aqueous fermentation broth or liquor, including cells. Cell density usually is expressed as gram/liter. Yield is defined as the amount of cells by weight produced for a given consumption by weight of carbon energy source or substrate fed to the ferment, and is usually expressed in grams/gram.

Accordingly, the invention relates to a continuous process of producing a single cell protein material which comprises culturing under aerobic fermentation conditions in aqueous mineral salts ferment at least one yeast species employing effective yeast culturing fermentation conditions, including amounts of carbon energy substrate, assimilable nitrogen source, make-up water, oxygen and aqueous mineral salts medium comprising a primary mineral salts medium and a trace mineral salts medium and continuously withdrawing aqueous ferment for recovering the resulting microorganisms as a single cell protein material, wherein said carbon energy substrate is selected from carbohydrates, alcohols, ketones, aldehydes, acids, and esters of 1 to 20 carbon atoms per molecule, and normal paraffins of 10 to 20 carbon atoms per molecule, which process is characterized by feeding said mineral salts to said aqueous ferment at such a rate to maintain in said aqueous ferment the following elements in at least the designated weights per liter of aqueous ferment: P-1.9 g, K-1 g, Mg-0.15 g, Ca-0.06 g, S-0.1 g, Fe-6 mg, Zn-2 mg, Cu-0.6 mg, and Mn-0.6 mg, thereby maintaining a cell density in said aqueous ferment of at least 60 and up to 160 grams, on a dried basis, per liter of aqueous ferment.

The high cell densities obtainable by the inventive process significantly streamline and reduce the cost of single cell protein production. Concentrating the single cell protein product by such as centrifuge means in many instances is sharply reduced or even eliminated. The cellular product can be, if desired, washed in washing means to remove residual unconsumed salts, and sent directly to a dryer means such as a spray dryer. The washings contain most of the remaining mineral salts not incorporated into the cells, and can be recycled to the fermentor. Requirements for water to the fermentation step thus are reduced considerably, and, importantly, there is little or no waste water requiring disposal. Alternatively, if desired, the total ferment including residual salts can be dried.

Heretofore, continuous processes for the production of single cell protein materials from yeast cultures typically gave ferment effluents of relatively low yeast cell contents, such as 20 to 25 grams of cells per liter of ferment. The invention however, provides a process whereby fermentation produces yeast cells at relatively very high levels, such as above 100 grams per liter of ferment. Such high cell densities in the ferment, coupled with high yields of yeast cells, mean more efficient effective production, particularly under continuous production conditions.

Detailed Disclosure of the Invention

In accordance with the invention, yeasts are grown under substantially continuous aerobic aqueous fermentation conditions on a suitable carbon energy source as substrate, employing effective amounts of molecular oxygen-containing gases, assimilable-nitrogen source, nutrient mineral salts, and, where necessary, adding additional nutrient organic material such as vitamins such as biotin and/or thiamine. In the inventive process the mineral salts are added at high levels such as will be described hereinafter, resulting in a fermentation process operating at high cell densities, with high yields. The invention essentially lies in almost the force-feeding of the cells by adding highly concentrated (relatively) mineral salts into the ferment, resulting in high growth rates. The mineral salts concentration in the liquid supernatant of the ferment (that is, the ferment excluding cells) itself remains, of course, at relatively low levels, since the salts are consumed by the cells in growth and reproduction. The salts concentration in the cells plus liquid supernatant, thus, is very high.

Fermentation Conditions

Culturing is accomplished in a growh medium comprising an aqueous mineral salts medium, the carbon energy source material, molecular oxygen, assimilable nitrogen, and of course, a starting inoculum of one or more particular species of yeast microorganisms to be employed.

In the invention high concentrations of mineral salts are fed to the ferment and high concentrations are maintained in the ferment. It is necessary to supply suitable amounts in proper proportions of selected mineral nutrients in the feed media, in order to assure proper microorganism growth, to maximize assimilation of the carbon and energy source by the cells in the microbial conversion process, and to achieve maximum cellular yields with maximum cell density in the fermentation media.

Although the composition of the ferment can vary over a wide range, depending in part on the yeast and substrate employed, the minerals content in the ferment (that is, liquid plus cells) in accordance with the invention is relatively high, at higher levels than heretofore considered suitable or practiced by the prior art. Set forth in the table below are the minimum, broad and preferred ranges of concentrations of various elements in the ferment, the concentration being expressed as of the element, though it is recognized that all or part of each can be present in the form of a soluble ion, or in cases such as P are present in a combined form of some type such as phosphate. The amount of each element is expressed in grams or milligrams per liter of ferment (aqueous phase, including cells):

| Element | Weight of element per liter of ferment | | |
| | Minimum | Broad range | Preferred range |
| --- | --- | --- | --- |
| P | 1.9 g | 1.9—20 g | 2.2—10 g |
| K | 1 g | 1—20 g | 1.5—10 g |
| Mg | 0.15 g | 0.15—3 g | 0.3—1.2 g |
| Ca | 0.06 g | 0.06—1.6 g | 0.08—0.8 g |
| S | 0.1 g | 0.1—8 g | 0.2—5 g |
| Fe | 6 mg | 6—140 mg | 9—80 mg |
| Zn | 2 mg | 2—100 mg | 3—40 mg |
| Cu | 0.6 mg | 0.6—16 mg | 1—10 mg |
| Mn | 0.6 mg | 0.6—20 mg | 0.9—8 mg |

Sulfur desirably is employed in the form of sulfate. Some of the metals required are advantageously added in the form of a sulfate, so that the minimum concentrations of sulfur normally are exceeded. Any or all of the metals listed can be used or present as the sulfate. Preferably, the magnesium, calcium, iron, zinc, copper, and manganese are employed in the form of a sulfate or chloride, or in the form of a compound which is converted in situ to a sulfate or chloride. The potassium preferably is employed as a sulfate, chloride or phosphate or in the form of a compound which is converted in situ to a sulfate, chloride, or phosphate. The phosphorus preferably is employed in the form of phosphoric acid or in the form of a

4

phosphate, monohydrogen phosphate, or dihydrogen phosphate, e.g., as a potassium or ammonium salt, or as a compound which is converted in situ to such a salt.

Other elements which may be present, at least in trace amounts, include such as sodium and cobalt, e.g., as a halide or sulfate; molybdenum, e.g., as molybdate, boron, e.g., as borate; selenium, e.g., as selenite or selenate; or iodine, e.g., as iodide.

In typical high cell density fermentation, the ferment will comprise about one-half supernatant medium and one-half cells, by volume. These one-half by volume cells, however, will contain at least about two-thirds of the mineral salts content of the ferment.

Yeast

According to the invention there is employed a culture of a yeast suitable for growth on carbon-containing substrates under aqueous fermentation conditions. Suitable yeasts include species from the genera *Candida, Hansenula, Torulopis, Saccharomyces, Pichia, Debaryomyces,* and *Brettanomyces.* The preferred genera include *Candida, Hansenula, Torulopsis, Pichia,* and *Saccharomyces.* Examples of suitable species include:

*Brettanomyces petrophilium*
*Candida boidinii*
*Candida lipolytica*
*Candida mycoderma*
*Candida utilis*
*Candida stellatoidea*
*Candida robusta*
*Candida claussenii*
*Candida rugosa*
*Candida tropicalis*
*Debaryomyces hansenii*
*Hansenula minuta*
*Hansenula saturnus*
*Hansenula californica*
*Hansenula mrakii*
*Hansenula silvicola*
*Hansenula polymorpha*
*Hansenula wickerhamii*
*Hansenula capsulata*
*Hansenula glucozyma*
*Hansenula henricii*
*Hansenula nonfermentans*
*Hansenula philodendra*
*Hansenula farinosa*
*Pichia farinosa*
*Pichia polymorpha*
*Pichia membranaefaciens*
*Pichia pinus*
*Pichia pastoris*
*Pichia trehalophila*
*Saccharomyces cerevisiae*
*Saccharomyces fragilis*
*Saccharomyces rosei*
*Saccharomyces acidifaciens*
*Saccharomyces elegans*
*Saccharomyces rouxii*
*Saccharomyces lactis*
*Torulopsis sonorensis*
*Torulopsis candida*
*Torulopsis bolmii*
*Torulopsis versatilis*
*Torulopsis glabrata*
*Torulopsis molishiana*
*Torulopsis nemodendra*
*Torulopsis nitratophilia,* and
*Torulopsis pinus.*

If desired, mixtures of two or more species of yeasts can be employed. The particular yeast employed depends in part on the carbon-containing substrate to be used since it is well known that different yeasts often require somewhat different substrates for best growth. For example, it is recognized that some particular strains of species listed above, such as of *Pichia pastoris*, do not grow on methanol.

Preferred are those *Pichia pastoris* and *Hansenula polymorpha* which do grow suitably on carbon energy feedstocks, particularly a lower alcohol such as methanol. Preferred are the particular novel strains designated as, or which are derived from, the strains deposited as *Pichia pastoris* NRRL Y—11431, *Pichia pastoris* NRRL Y—1140, *Hansenula polymorpha* NRRL Y—11170, and *Hansenula polymorpha* NRRL Y—11432, since these strains have been found to be particularly suitable for use in producing SCP protein materials at high cell densities with high yields. This feature is considered particularly unusual for these species since out of four *Pichia pastoris* cultures tested, only two would grow on methanol at all.

The carbon energy substrate can be any of carbohydrates, alcohols, ketones, aldehydes, acids and esters of 1 to 20 carbon atoms, and normal paraffins of 10 to 20 carbon atoms. It is recognized that particularly yeasts do vary in their preference for various substrates.

The preferred substrates for aqueous fermentation conditions are the carbon-oxygen-hydrogen significantly water-soluble compounds. The term "oxygenated hydrocarbons" includes the water-soluble carbohydrates, as well as those alcohols, ketones, esters, acids and aldehydes, and mixtures, which are reasonably water-soluble in character. The more suitable oxygenated hydrocarbons usually are those of substantially greater water-solubility of up to 10 carbon atoms per molecule, or are the water-soluble carbohydrates generally.

Exemplary carbohydrates include glucose, fructose, galactose, lactose, sucrose, starch, dextrin, and the like, alone or in admixture. Of the other types of oxygenated hydrocarbons, examples include methanol, ethanol, ethylene glycol, propylene glycol, 1-propanol, 2-propanol, glycerol, 1-butanol, 2-butanol, 3-methyl-1-butanol, 1-pentanol, 2-hexanol, 1,7-heptanediol, 1-octanol, 2-decanol, 1-hexadecanol, 1-eicosanol, acetone, 2-butanone, 4-methyl-2-pentanone, 2-decanone, 3-pentadecanone, 2-eicosanone, formaldehyde, acetaldehyde, propionaldehyde, butyraldehyde, hexanal, 7-merthyloctanal, tetradecanal, eicosanal, acetic acid, propionic acid, butyric acid, glutaric acid, 5-methylhexanoic acid, azelaic acid, dodecanoic acid, eicosanoic acid, methyl formate, methyl acetate, ethyl acetate, propyl butyrate, isopropyl hexanoate, hexyl 5-methyloctanoate, octyl dodecanoate, and the like, as well as mixtures thereof.

The said normal paraffins having from 10 to 20 carbon atoms per molecule are much less preferred because of the sometimes difficulty in removing residual substrate from the single cell protein cells. Typical examples include decane, undecane, dodecane, tridecane, tetradecane, pentadecane hexadecane, octadecane, eicosane, and mixtures thereof.

Preferred are the water-soluble alcohols of 1 to 4 carbon atoms, water-soluble acids from 2 to 4 carbon atoms, and the water-soluble carbohydrates. Preferred are the water-soluble monohydric aliphatic hydrocarbyl alcohols. It should be noted that 2-methyl-1-propanol is inhibitory to some yeasts, and in fermentations with such yeasts this alcohol should be avoided. Most preferred are the alcohols of 1 to 4 carbon atoms (other than 2-methyl-1-propanol); of these methanol and ethanol are preferred over the others; and methanol is the most preferred due to the low relative cost of such feedstock.

Petroleum gases can be oxidized, and the water-soluble materials employed, such as oxidation of methane, ethene, and the like to provide mixtures predominantly of the corresponding alcohol as well as various aldehydes, ketones, acids, and the like, and similarly suitable hydrocarbon fractions from various petroleum refinery sources produced within the integrated refining and chemical processing complex, sometimes termed a petrocomplex, can be utilized for fermentation purposes.

The salts in the supernatant are at a relatively low concentration, since there is a high take-up by the growing reproducing cells. The mineral salts in the cells may not be as fed or applied since some may be in a bound organic form. Mineral analysis of the ferment, of course, would reflect a total mineral content.

In addition to the mineral salts, vitamins (organic growth factors) can be employed in the ferment as is known in the art, where their presence is desirable for the propagation of the particular yeast chosen. For example, many yeasts for their proper propagation, seem to require the presence of one or both of the vitamins biotin and thiamine, or other medium constituents which contain these vitamins, e.g., yeast extract. Thus, for example, with a yeast such as a *Hansenula polymorpha*, it is desirable to employ biotin in an amount of 0.04 to 0.8 milligram per liter of aqueous mineral medium and thiamine hydrochloride in an amount of 4 to 80 milligrams per liter of aqueous mineral medium. Alternatively, all or part of the biotin and thiamine can be provided by use of yeast extract or the like.

It was found earlier that the employment of water containing residual amounts of chlorine, such as is commonly encountered in water for purification treating processes in some countries, in preparing mineral medium to which growth factors are added for use in aqueous aerobic fermentation processes tends to render ineffective the growth factors, particularly vitamins such as biotin or thiamine. It was also found that in such fermentation system, employing an aqueous mineral medium prepared from water containing residual chlorine, that removal of the chlorine befor eadding the growth factors avoids the loss or deactivation of the growth factors. Thus, there have been found methods of treating the residual chlorine-containing water so as to effectively eliminate the traces of residual chlorine therefrom and thus avoid the vitamin loss.

It has now been found that water containing heretofore objectionable amounts of residual chlorine nevertheless can be utilized in fermentation processes employing vitamins yet without inactivation of the vitamins by the chlorine if the vitamins are added to the fermentation zone as a separate stream from the aqueous nutrient medium. Thus, the mineral nutrient medium can now employ the water containing trace amounts of chlorine. This arrangement thus avoids the need for pretreating, by expensive and/or time consuming methods, the water which contains residual trace amounts of chlorine.

The above described separate addition of the vitamins to the fermentation zone is preferably and conveniently accomplished by admixing the vitamins with at least a portion of but preferably the entire carbon energy substrate stream prior to charging these materials to the fermentation zone. If an aqueous admixture of vitamins and carbon energy substrate is employed, the water used for initial dilution of the vitamins should preferably be free of traces of residual chlorine, such as deionized water, to avoid any premature loss before mixing with the aqueous carbon-substrate stream such as methanol-in-water.

If desired, and also preferred, an admixture can be made of water and a water-soluble carbon substrate such as methanol, such as about 20 volume percent methanol in water, and then the vitamins can be dissolved in the methanol-in-water solution, and fed then to the fermentor. By this mode, residual chlorine need not be first removed, but yet the vitamins are fully preserved.

In a more preferred embodiment, the separate addition of vitamins to the fermentation zone is accomplished utilizing an admixture of vitamins, at least a portion of the carbon energy substrate as noted above and the further addition of an aqueous trace mineral salts solution. The trace mineral salts comprise what has been referred to hereinabove as the trace elements such as cobalt, molybdenum, boron, selinium, iodine, as well as manganese, copper, zinc, and iron. The use of this more preferred embodiment not only avoids the vitamin inactivation problem caused by traces of chlorine in the water used for the aqueous mineral salts medium, but also avoids another problem that is often encountered in the fermentation processes. This problem is the formation of precipitates, in the heat sterilization zone employed to treat the aqueous mineral salts medium, requiring frequent cleaning. The presence of the trace mineral salts in its usual admixture with the primary mineral nutrient salts apparently promotes the formation of troublesome precipitates in the heat sterilization zone. Thus, by not including the trace mineral salts in the aqueous mineral salts medium stream, but rather instead of charging the trace mineral salts in admixture with the vitamins and at least a portion of the carbon energy substrate solves two very troublesome problems. As noted above, the water used to prepare the admixture of trace mineral salts, a least a portion of the carbon energy substrate, and the vitamins should preferably be free of residual traces of chlorine.

The stream comprises of vitamins, a portion of the carbon energy substrate, and trace minerals can be sterilized by filtration if desired. However, it is preferable and convenient to combine said stream with the major carbon energy substrate stream prior to charging to the fermentation zone and filtering the entire combined streams just prior to charging to the fermentation zone.

The fermentation itself is an aerobic process requiring molecular oxygen which is supplied by a molecular oxygen-containing gas such as air, or even substantially pure molecular oxygen, to maintain the ferment with an oxygen partial pressure effective to assist the microorganism species in growing in a thriving fashion. By using an oxygenated hydrocarbon substrate, the total oxygen requirements for growth of the microorganism are reduced from the requirements when a paraffin is used. Even so, adequate quantities of molecular oxygen must be supplied for growth, since the assimilation of the substrate and corresponding growth of the microorganisms is, in part, a combustion process.

The rate at which the molecular oxygen is supplied to the ferment should be such that the growth of the yeast is not limited by lack of oxygen. Fermentor designs vary widely in their ability to transfer oxygen to the culture. Although the overall aeration rates can vary over a considerable range, with fermentors that are very efficient in oxygen transfer aeration generally is conducted at a rate of 0.5 to 8, preferably 1 to 6, volumes (at the pressure employed and at 25°C) of molecular oxygen-containing gas per liquid volume in the fermentor per minute. This amount is based on air of normal oxygen content being supplied to the reactor, and in terms of pure molecular oxygen the respective ranges would be 0.1 to 1.7, or preferably 0.2 to 1.3, volumes (at the pressure employed and at 25°C) of molecular oxygen per liquid volume in the fermentor per minute.

The pressure employed for the microbial fermentation step can range widely. Typical pressures are O to 150 psig (0 to 1.03 MPa gauge) preferably O to 60 psig (0 to 0.42 MPa gauge), more preferably at least slightly over atmospheric pressure, as a balance of equipment and operating costs versus oxygen solubility achieved. Greater than atmospheric pressures are advantageous in that such pressures do tend to increase the dissolved oxygen concentration in the aqueous ferment, which in turn can help increase cellular growth rates. At the same time this is counterbalanced by the fact that high pressures do increase equipment and operating costs.

The fermentation temperature can vary somewhat, but generally will be 25°C to 65°C, generally preferably 28°C to 50°C. The yeast cultures *Pichia pastoris* Culture 21-2 deposited as NRRL Y-11431 and *Pichia pastoris* Culture 21-1 deposited as NRRL Y-11430 generally prefer a ferment temperature of about 30°C. The *Hansenula polymorpha* NRRL Y-11170 and *Hansenula polymorpha* Culture 21-3 deposited as NRRL Y-11432 appear to prefer a ferment temperature on the order of about 38°C to 40°C.

Yeasts require a source of assimilable nitrogen. The assimilable nitrogen can be supplied by any nitrogen-containing compound or compounds capable of releasing nitrogen in a form suitable for metabolic utilization by the yeast microorganism. While a variety of organic nitrogen source compounds, such as protein hydrolysates, technically can be employed, usually cheaper nitrogen-containing compounds such as ammonia, ammonium hydroxide, urea, and various ammonium salts such as

7

ammonium phosphate, ammonium sulfate, ammonium pyrophosphate, and ammonium chloride can be utilized. Ammonia gas itself is convenient for large scale operations, and can be employed by bubbling through the aqueous microbial ferment in suitable amounts. At the same time, such ammonia also assists in pH control.

The pH range in the aqueous microbial ferments should be in the range of 3 to 7, more preferably and usually 3.5 to 5.5. Preferences of certain microorganisms for a pH range are dependent to some extent on the medium employed, as well as on the particular microorganism, and thus may change somewhat with change in medium as can be readily determined by those skilled in the art.

The average retention time of the ferment in the fermentor can vary considerably, depending in part on the fermentation temperature and yeast culture employed. Generally, the retention time will be 2 to 30 hours, preferably 4 to 14 hours, based on average retention.

High concentrations of some of the described carbon and energy substrates, particularly such as methanol or formaldehyde or the like, may be inhibitory to satisfactory microbial growth or even toxic to the microorganisms in the fermentation. Relatively high concentrations of substrates thus should be avoided, so that it is generally desirable to maintain the substrate concentration in the ferment at a maximum tolerable level. With some of the lower alcohols, this level in the ferment generally is 0.001 to 5 volume percent, preferably 0.01 to 0.05 volume percent, while with the aldehydes the level should be one-tenth of these due to the toxicity of aldehydes, so as to neither starve nor inhibit the growth rates of the microorganisms chosen.

When the carbon and energy source material contains an aldehyde in amounts potentially deleterious to the microorganism, the deleterious aldehyde effects can be alleviated by first treating the substrate with a suitable amount of a nitrogen-containing compound, preferably ammonia, ammonium hydroxide, or other active ammonium compound, in a ratio of 0.01 to 10 mol equivalents of such nitrogen-containing compounds per mol of aldehyde. Such a treated substrate then is not only the carbon energy source, but also contains at least a portion of the necessary assimilable nitrogen.

Conveniently, the fermentation is conducted in such a manner that the carbon-containing substrate can be controlled as a limiting factor, thereby providing good conversion of the carbon-containing substrate to yeast cells and avoiding potential contamination of the yeast cells with a substantial amount of uncoverted substrate. The latter is not a problem with water-soluble substrates, since any remaining traces are readily washed off. It may be a problem, however, in the case of non-water-soluble substrates such as the higher n-paraffins, requiring added product treatment steps such as removal of residual hydrocarbon by suitable solvent washing steps.

Continuous operation is much to be preferred for ease of control, production of uniform quantities of uniform products, and most economical uses of all equipment. In a continuous process, the carbon and energy source material as substrate, aqueous mineral medium, assimilable nitrogen source, and molecular oxygen-containing gases, are added continuously to the ferment in the fermentor combined with continuous withdrawal of ferment. Although the volume ratio of added carbon energy substrate: added aqueous mineral medium can vary over a wide range, depending in part on the nature of the carbon-containing substrate, generally it will be in the range of 1:9 to 6:4, preferably in the range of 2:8 to 5:5.

If desired, part or all of the carbon energy source material and/or part of the assimilable nitrogen source such as ammonia can be added to the aqueous mineral medium prior to passing the aqueous mineral medium to the fermentor. Most convenient in my work in high cell density fermentations has been the use of a feed ratio of about 40 volume percent alcohol to 60 volume percent mineral salts medium.

Each of the streams introduced into the reactor preferably is controlled at a predetermined rate, or in response to a need determinable by monitoring, such as concentration of the carbon and energy substrate, pH, dissolved oxygen, oxygen or carbon dioxide in the off-gases from the fermentor, cell density measurable by light transmittancy, or the like. The feed rates of the various materials can be varied so as to obtain as rapid a cell growth rate as possible, consistent with efficent utilization of the carbon and energy source, to obtain as high a yield of yeast cells relative to substrate charge as possible. Thus, by the process of the invention, yeast cells can be obtained in yields of 30 to 110 grams per 100 grams substrate charged, depending in part on the particular substrate used.

All the equipment, reactor, or fermentation means, vessel or container, piping, attendant circulating or cooling devices, and the like, most preferably are sterilized, usually employing steam such as at 250°F (121°C) for at least 15 minutes. The sterilized reactor is inoculated with a culture of the specified microrganism in the presence of all the required nutrients, including molecular oxygen, and the carbon-containing substrate.

The type of fermentor employed is not critical in the practice of the fermentation process of the invention, though presently preferred is operation in a foam-filled fermentor. A fermentor designed to encourage and maintain the produced foam is beneficial to the process of achieving the increased oxygen transfer necessary to maintain desired high cell densities and rapid growth rates.

In starting out a fermentation, the aqueous mineral medium suitable concentration of carbon energy source, assimilable nitrogen, trace components where desired, and the starting innoculum of yeast are placed in a sterilized fermentor, and suitable flows of oxygen and the various feeds are gradually commenced. If desired, the initial fermentation substrate can be such as glucose, with gradual change to such a methanol as cell density builds up. It is possible to being at low mineral salts levels in the aqueous

EP 0 017 853 B2

ferment and build up to a high mineral salts level by feeding an aqueous mineral medium having a high concentration of mineral salts to the ferment, though normally high salts medium is simply added initially to the fermentor to commence immediate operation. One skilled in the art realizes that a brief lag time will usually occur at start up before the innoculum builds up enough cells for full input of salts and substrate to be utilized.

Product Recovery

The yeast cells produced in accordance with the high cell density process are recovered from the fermentation admixture effluent by conventional means, such as by centifugation or filtration. If desired, extracellular products can be recovered from the substantially cell-free remaining supernatant liquid by conventional means. The substantially cell-free effluent can be treated, for example, with acetone or a lower alcohol such as methanol or ethanol to precipitate any polymeric material produced extra-cellularly. The cell-free effluent also can be treated by solvent extraction and/or base extraction to recover, if desired, other extra-cellular products such as pigments, vitamins, or organic acids produced during the culturing process. The cell-free effluent, with or without such intervening treatment, can be returned to the fermentor as a part of the aqueous makeup or as a substantial or almost total part of the aqueous makeup, to avoid waste disposal problems insofar as possible.

The microbial cells usually are killed by heat or chemical means, and this can be done before or after the separation of the cells from the fermentor effluent. The yeast cells are a valuable source of protein for man as well as beast. For human consumption, the cells can be treated as necessary to reduce the nucleic acid, but for animal feed purposes such treatment does not appear presently necessary.

In accordance with the invention, employing high cell density operation, e.g., a cell density within the range of 60 to 160, preferably 70 to 150, grams of yeast cells, on a dried basis, per liter of fermentation admixture, can be obtained in high yield. If desired, the cells can be recovered from the fermentation admixture by centrifugation or other separation means. Also, if desired, the concentrated cells then can be washed such as by mixing with water, and separated such as by recentrifuging, or by adding water prior to or during centrifugation to substantially free the cells of mineral medium, and the washings including the separated mineral medium then can be returned to the fermentor as water and mineral medium make-up, thus substantially reducing or avoiding waste disposal problems. The recovered cells then be simply dried to produce a dried product for future use. If desired, the high cell density fermentor effluent in total can be dried to produce a whole dried product of dried cells and residual water soluble substances including salts, and this whole-dried product used as a very useful animal feed of high protein-high salts character.

## Examples

The following are descriptive runs employing the process in accordance with the invention. Particular amounts of materials, or particular types of feedstocks employed, particular species or strains of yeast, should be considered as illustrative and not as limitative.

## Example I

In a run conducted under continuous aerobic fermentation process conditions, methanol and an aqueous mineral salts medium in a volume ratio of 30.15 to 69.85, respectively, were fed individually to a fermentor inoculated with the yeast *Pichia pastoris* Culture 21-2 deposited as NRRL Y-11431. No preconditioning medium or substrate was employed. The fermentor was a 4-filter fermentor with a 2-liter liquid volume, with automatic pH, temperature, and level control. Agitation was provided by two impellers rotating at 1000—1200 rpm. The aeration rate was 1—1.5 volumes (at about atmospheric pressure and about 25°C) per volume of ferment per minute of air supplemented with and including sufficient oxygen to maintain in the fermentation mixture an amount of dissolved oxygen equal to about 20 percent of that which would be dissolved in the fermentation mixture saturated with air at atmospheric pressure and about 30°C. Aqueous ammonium hydroxide (from 2 parts concentrated ammonium hydrate and 1 part deionized water, by volume) was added at such a rate as to maintain the pH of the fermentation mixture at about 3.5.

The aqueous mineral salts medium employed was prepared by mixing, for each liter of solution 12.5 ml 85 percent $H_3PO_4$, 2.5 g 85 percent KOH, 8.5 g KCl, 7.0 g $MgSO_4.7H_2O$, 1.5 g $CaCl_2.2H_2O$. 25 ml of trace mineral solution A, 25 ml of trace mineral solution B, 10 ml of a biotin-thiamine hydrochloride solution about 0.08 ml of antifoam agent (Mazu $^{(TM)}$ DF-37C), and sufficient deionized water to make 1 liter of solution.

Trace mineral solution A was prepared by mixing, for each liter of solution, 4.8 g $FeCl_3.6H_2O$, 2.0 g $ZnSO_4.7H_2O$, 0.02 g $H_3BO_3$, 0.20 g $Na_2MoO.2H_2O$, 0.30 g $MnSO_4.H_2O$, 0.08 g KI, 0.06 g $CuSO_4.5H_2O$. 3 ml conc. $H_2SO_4$, and sufficient deionized water to make 1 liter of solution.

Trace mineral solution B was prepared by mixing for each liter of solution, 2.0 g $FeCl_3.6H_2O$, 2.0 g $ZnSO_4.7H_2O$, 0.3 g $MnSO_4.H_2O$, 0.6 g $CuSO_4.5H_2O$, 2 ml conc. $H_2SO_4$, and sufficient deionized water to make 1 liter of solution.

The biotin-thiamine hydrochloride solution was prepared by mixing 2 mg biotin, 200 mg thamine hydrochloride, and 50 ml deionized water.

The fermentation was conducted at about 30°C and about atmospheric pressure, with a retention time of 7.0 hours.

9

Yeast cells were separated from the fermentation effluent by centrifugation, washed by suspension in water and recentrifugation, dried overnight at 100C, and weighed. On a dried basis, the yeast cells were produced in a yield of 42.3 g per 100 g of methanol fed, the cell density being at the high level of 100.7 g of cells per liter of effluent.

Example II

A further fermentation run was conducted using essentially the procedure described in Example I except that the composition of the aqueous mineral salts medium was somewhat different, the volume ratio of methanol to the aqueous mineral salts mediums was 40.8 go 59.2, the aqueous ammonium hydroxide for pH control was prepared from 3 parts concentrated ammonium hydroxide and 1 part deionized water, by volume, and the fermentation retention times was 8.35 hours.

The aqueous mineral salts medium for use in this run was prepared by mixing, for each liter of solution, 20.0 ml 85 percent $H_3PO_4$, 4.0 g 85 percent KOH, 12.0 g kcl, 10.4 g $MgSO_4.7H_2O$, 2.4 g $CaCl_2.2H_2O$, 40 ml of the trace mineral solution A as described in Example I, 40 ml of the trace mineral solution B as described in Example I, 16 ml of the biotin-thiamine hydrochloride solution as described in Example I, about 0.08 ml of antifoam agent, and sufficient deionized water to make 1 liter of solution.

Yeast cells were separated from the fermentation effluent, washed, and dried as in Example I. On a dried basis, the yeast cells were produced in a yield of 41.4 g per 100 g of methanol fed, the cell density being at the very desirable high level of 133.3 g of cells per liter of effluent.

Example III

A continuous aerobic fermentation process was conducted in the fermentor as described in Example I, this time inoculated with the yeast species *Hansenula polymorpha* Culture 21-3 deposited as NRRL Y-11432. To the fermentor was fed a mixture of methanol and aqueous mineral salts medium containing 300 ml methanol per liter total solution. The stirred fermentation mixture was aerated by passing into the fermentor 2 volumes (at about atmospheric pressure and about 25°C) per volume of ferment per minute of air supplemented with and including sufficient oxygen to maintain in the fermentation mixture an amount of dissolved oxygen equal to about 20 percent of that which would be dissolved in the fermentation mixture saturated with air at a atmospheric pressure and about 38°C.

Aqueous ammonium hydroxide (from 2 parts concentrated ammonium hydroxide and 1 part deionized water, by volume) was added at a rate to maintain the pH of the fermentation mixture at 3.7 to 4.1.

The mixture of methanol and aqueous mineral salts medium was prepared by mixing, for each liter of solution, 300 ml methanol, 6 ml 85 percent $H_3PO_4$, 3 g KCl, 4.5 g $MgSO_4.7H_2O$, 0.6 g $CaCl_2.2H_2O$, 0.3 g NaCl, 10 ml of trace mineral solution A as described in Example I, 10 ml of trace mineral solution B as described in Example I, 4 ml of the biotin-thiamine hydrochloride solution described in Example I, 4 drops of antifoam agent, and sufficient deionized water to make 1 liter of solution.

The fermentation was conducted at about 38°C and about atmospheric pressure, with a retention time of 5.66 hours.

Yeast cells were separated from the fermentation effluent, washed, and dried as in Example I. On a dried basis, the yeast cells were produced in a yield of 31.0 g per 100 g of methanol fed, the cell density being at 73.3 g of cells per liter of effluent.

Example IV

In a continuous aerobic fermentation process, methanol and an aqueous mineral medium in a volume ratio of 36.9 go 63.1, respectively, were fed individually to a fermentor inoculated with the yeast species *Pichia pastoris* Culture 21-1 deposited as NRRL Y-11430. The fermentor was a 1500-liter foam-filled fermentor with a liquid volume of about 600 liters, with automatic pH, temperature, and level control, and equipped with a draft tube. Agitation was provided by a turbine, below the draft tube, driven at 750 rpm. The aeration rate was about 1.6 volumes of air (at about 38 psig (262 kPa gauge) and about 25°C) per volume of ferment in the fermentor per minute. Anhydrous ammonia was added at a rate to maintain the pH of the ferment mixture at about 3.5.

The primary aqueous mineral salts medium was prepared by mixing, for each liter solution, 12.2 ml 75 percent $H_3PO_4$, 6.0 g KCl, 6.0 g $MgSO_4.7H_2O$, 0.8 g $CaCl_2.2H_2O$, 2.0 g 85 percent KOH, 2.0 ml of trace mineral solution C, 0.8 ml of a biotin-thiamine hydrochloride solution and sufficient tap water to make 1 liter of solution, the tap water first having been treated with enough sodium thiosulfate to react with the free chlorine present therein.

Trace mineral solution C was prepared by mixing, for each liter of solution, 65 g $FeCl_3.6H_2O$, 18 g $ZnSO_4.7H_2O$, 5.0 g $MnSO_4.H_2O$, 6.0 g $CuSO_4.5H_2O$, 2.0 ml conc. $H_2SO_4$, and sufficient deionized water to make 1 liter of solution.

The biotin-thiamine hydrochloride solution was made by mixing the components in the ratio of 0.4 g biotin to 40 g thiamine hydrochloride to 1 liter deionized water.

The fermentation was conducted at 30°C and 38 psig pressure, with a retention time of 12.7 hours.

Yeast cells were separated from the fermentation effluent by centrifugation, washed by suspension in water and recentrifugation, dried overnight at 100°C, and weighed on a dried basis, the yeast cells were produced in a yield of 37 g per 100 g of methanol fed, the cell density being a very desirable 110.3 g of cells per liter of effluent.

## Example V

In a continuous aerobic fermentation process, methanol and an aqueous mineral salts medium in a volume ratio of 40 to 60, respectively, were fed individually to a fermentor inoculated with the yeast species *Pichia pastoris* Culture 21-1 deposited as NRRL Y-11430. The fermentor was a 1500-liter foam-filled fermentor with a liquid volume of about 610 liters, with automatic pH, temperature, and level control. Agitation was provided by two conventional paddle-type turbines driven at 1000 rpm. The aeration rate was about 4 volumes of air (at about 38 psig (262 kPa gauge) and about 25°C) per volume of ferment in the fermentor per minute. Anhydrous ammonia was added at such a rate as to maintain the pH of the fermentation mixture at about 3.5.

The aqueous mineral salts medium was prepared by mixing, with each liter of tap water, 15.86 ml 75 percent $H_3PO_4$, 9.53 g $K_2SO_4$, 7.8 g $MgSO_4.7H_2O$, 0.6 g $CaCl_2.2H_2O$, and 2.6 g 85 percent KOH. The trace mineral solution plus biotin was fed separately via the methanol stream at a rate of 10 ml per liter of methanol. The trace mineral solution plus biotin was prepared by mixing 780 ml trace mineral solution D. 20 ml water, 200 ml methanol and 0.032 g biotin.

Trace mineral solution D was prepared by mixing, for each liter of solution, 65 g $FeSO_4.7H_2O$, 20 g $ZnSO_4.7H_2O$, 3.0 g $MnSO_4.H_2O$, 6.0 g $CuSO_4.5H_2O$, 5.0 ml conc. $H_2SO_4$, and sufficient deionized water to make 1 liter of solution.

The aqueous mineral salts medium was fed at a rate of 31.5 liters per hour and the methanol at a rate of 21 liters per hour.

The fermentation was conducted at 30°C and about 38 psig pressure, with a retention time of 11.6 hours.

For analytical purposes yeast cells were separated from the fermentation effluent by centrifugation, washed by suspension in water and recentrifugation, dried overnight at 100°C, and weighed. On a dried basis, the yeast cells were produced in a yield of 40.6 g per 100 g of methanol fed, the cell density being a very desirable 128.4 g of cells per liter of effluent. The total solids content of the ferment (effluent from the fermentor) was 134.7 g per liter, cells plus dissolved solids. The effluent from the fermentor was fed directly through a pasteurizer to kill the yeast and into a spray dryer without further concentration of treatment.

## Example VI

In a continuous aerobic fermentation process, methanol and an aqueous mineral salts medium in a volume ratio of 29 to 71, respectively, were fed individually to a fermentor inoculated with the yeast species *Hansenula polymorpha* NRRL Y-11170. The fermentor was 1500-liter foam-filled fermentor with a liquid volume of about 560 liters, with automatic pH, temperature, and level control and equipped with a draft tube. Agitation was provided by a turbine, below the draft tube driven at 810 rpm. The aeration rate was about 5 volumes of air (at about 38 psig (262 kPa gauge) and about 25°C) per volume of ferment in the fermentor per minute. Anhydrous ammonia was added at such a rate as to maintain the pH of the fermentation mixture at about 3.5.

The aqueous mineral salts medium was prepared by mixing 10.38 ml 75 percent $H_3PO_4$, 4.29 g KCl, 6.44 g $MgSO_4.7H_2O$, 0.86 g $CaCl_2.2H_2O$, 0.43 g NaCl, 3.0 ml of trace mineral solution C, and 0.64 ml of a biotin-thiamine hydrochloride solution in 1000 ml tap water, the tap water first having been treated with enough solid thiosulfate to react with the free chlorine present threin.

The fermentation was conducted at 39—40°C and 38 psig (262 kPa gauge) pressure, with a retention time of 7.6 hours.

For analysis yeast cells were separated from the fermentation effluent by centrifugation, washing by suspension in water and recentrifugation, washing by suspension in water and recentrifugation, dried overnight at 100°C, and weighed. On a dried basis, the yeast cells were produced in a yield of 33.3 g per 100 g of methanol fed, the cell density being a desirable 76.2 g of cells per liter of effluent.

Novel Yeasts

Discovery of yeast with the capability of rapid growth and high productivity rates is distinctly advantageous.

There have been discovered three very unique cultures of yeasts, namely. *Picia pastoris* NRRL Y-11431, *Pichia pastoris* NRRL Y-11430, and *Hansenula polymorpha* NRRL Y-11432, with highly desirable and useful properties. These unique cultures grow particularly well at higher mineral salts levels and cell densities.

These three unique cultures of yeasts grow effectively with high productivity on oxygenated hydrocarbon feedstocks, particularly lower alcohols, most preferably methanol or ethanol. This is particularly noteworthy with regard to the new *Pichia pastoris* cultures since some cultures of the species *Pichia pastoris* simply cannot grow on methanol. These unique species are designated as follows:

| Culture name | Strain Designation | Depository Designation |
|---|---|---|
| *Pichia pastoris* | 21-2 | NRRL Y-11431 |
| *Pichia pastoris* | 21-1 | NRRL Y-11430 |
| *Hansenula polymorpha* | 21-3 | NRRL Y-11432 |

The designations NRRL Y-11431, NRRL Y-11430, and NRRL Y-11432 reflect the fact that the novel yeast cultures 21-2, 21-1, and 21-3, have been deposited with the official depository, United States Department of Agriculture, Agricultural Research Service, Northern Regional Research Laboratory, Peoria, Illinois 61604, by depositing therein two agar slant cultures of each, and having received from the depository the individual NRRL strain designations as indicated.

These strain have been classified:

| | *Pichia pastoris*<br>Culture 21-1<br>NRRL Y-11430 | *Pichia pastoris*<br>Culture 21-2<br>NRRL Y-11431 | *Hansenula polymorpha*<br>Culture 21-3<br>NRRL Y-11432 |
|---|---|---|---|
| Division | Ascomytina | Ascomytina | Ascomytina |
| Class | Hemiascomycetes | Hemiascomycetes | Hemiascomycetes |
| Order | Endomycetales | Endomycetales | Endomycetales |
| Family | Saccharomycetaceae | Saccharomycetaceae | Saccharomycetaceae |
| Genus | Pichia | Pichia | Hansenula |

The novel and unique microorganisms can be futher characterized by properties as shown in the following tabulation:

| Culture<br>Property or<br>Test Result [a] | *Pichia pastoris*<br>Culture 21-1<br>NRRL Y-11430 | *Pichia pastoris*<br>Culture 21-2<br>NRRL Y-11431 | *Hansenula polymorpha*<br>Culture 21-3<br>NRRL Y-11432 |
|---|---|---|---|
| Gram straining | + | + | + |
| Spore forming | + | + | + |
| Aerobic | + | + | + |
| Approx. size, $\mu$ | 3—5 | 3—5 | 3—5 |
| Optimum temps., °C | 30 | 30 | 38—40 |
| Optimum pH | 3.5—5.5 | 3.5—5.5 | 3.5—5.5 |
| Growth factors | Biotin | Biotin | Biotin and thiamine |
| Cell appearance on malt extract agar | Colonies turn deep tan with formation of hat-shaped spores | Form hat-shaped spores | Form hat-shaped spores |
| Colony appearance on YM agar | Cream colored; no pseudomycelium | Cream colored; no pseudomycelium | Cream colored; no pseudomycelium |
| Assimilation of sugars | | | |
| Glucose | + | + | + |
| Galactose | W/L | | |
| L-Sorbose | — | | |
| Maltose | — | | |
| Sucrose | — | | |
| Cellobiose | — | | |
| Trehalose | — | | |
| Lactose | — | | |
| Melibiose | — | | |
| Raffinose | — | | |
| Melezitose | — | | |
| Inulin | — | | |
| Soluble starch | W/L | | |
| Xylose | — | | |
| L-arabinose | — | | |
| D-arabinose | — | | |
| D-Ribose | — | | |
| L-Rhamnose | — | | |
| Ethyl alcohol | + | + | + |
| Methyl alcohol | + | + | + |

| Culture Property or Test Result[a] | Pichia pastoris Culture 21-1 NRRL Y-11430 | Pichia pastoris Culture 21-2 NRRL Y-11431 | Hansenula polymorpha Culture 21-3 NRRL Y-11432 |
|---|---|---|---|
| Glycerol | + | | |
| Erthritol | − | | |
| Adonitol | − | | |
| Dulcitol | − | | |
| Mannitol | + | | |
| Sorbitol | + | | |
| Methyl-d-glucoside | − | | |
| Salicin | − | | |
| Inositol | − | | |
| Fermentation of sugars | | | |
| Glucose | + | + | + |
| Galactose | − | | |
| Sucrose | − | | |
| Lactose | − | | |
| Maltose | − | | |
| Raffinose | − | | |
| Trehalose | − | | |
| Nitrate Assimilation | − | | |
| Urease activity | − | | |
| Arbutin split | − | | |
| Pseudomycelium or hyphae formed on corn meal | − | | |
| on yeast agars | − | − | − |
| Guanine plus cytosine content of DNA | 40% | | |

+=positive
−=negative
W/L=weak to latent reaction
[a]=if value not shown, means not determined

Of course, as with all microorganisms, some of the characteristics may be subject to some variation depending on the medium and particular conditions.

The media recipies shown in the Examples can be used to culture the novel yeast species, though they will grow on other than methanol-containing substrates. These novel yeast cultures can be employed not only with high salts-media feeds as herein described, but also can be employed under conventional fermentations conditions using a medium such as:

Medium IM-1

| Component | Amount |
| --- | --- |
| $KH_2PO_4$ | 5.0 g |
| $MgSO_4 7H_2O$ | 0.5 g |
| $CaCl_2.2H_2O$ | 0.1 g |
| KCl | 0.5 g |
| $(NH_4)_2SO_4$ | 3.0 g |
| Biotin | 0.04 mg |
| Thiamine | 4.0 mg |
| Trace mineral solution[a] | 2.5 ml |
| Water | 1,000 ml |

Sterile methanol[b] to give   0.5—1.0 Vol%
[a] See recipe below.
[b] Added just prior to use.

Trace mineral solution

| Component | Amount |
| --- | --- |
| $CuSO_4.5H_2O$ | 0.06 g |
| KI | 0.08 g |
| $MnSO_4.H_2O$ | 0.3 g |
| $Na_2MoO_4.2H_2O$ | 0.2 g |
| $H_3BO_3$ | 0.02 g |
| $ZnSO_4.7H_2O$ | 2.0 g |
| $FeCl_3.6H_2O$ | 4.8 g |
| Distilled water | 1,000 ml |
| $H_2SO_4$ (conc.) | 3 ml |

**Claims**

1. A continuous process of producing a single cell protein material which comprises culturing under aerobic fermentation conditions in aqueous mineral salts ferment at least one yeast species employing effective yeast culturing fermentation conditions, including amounts of carbon energy substrate, assimilable nitrogen source, make-up water, oxygen and aqueous mineral salts medium comprising a primary mineral salts medium and a trace mineral salts medium and continuously withdrawing aqueous ferment for recovering the resulting microorganisms as a single cell protein material, wherein said carbon energy substrate is selected from carbohydrates, alcohols, ketones, aldehydes, acids, and esters of 1 to 20 carbon aoms per molecule, and normal paraffins of 10 to 20 carbon aoms per molecule, characterized by feeding said mineral salts to said aqueous ferment at such a rae to maintain in said aqueous ferment the

# EP 0 017 853 B2

following elements in at least the designated weights per liter of aqueous ferment P—1.9 g, K—1 g, Mg—0.15 g, Ca—0.06 g, S—0.1 g, Fe—6 mg, Zn—2 mg, Cu—0.6 mg, and Mn—0.6 mg, thereby maintaining a cell density in said aqueous ferment of at least 60 and up to 160 grams, on a dried basis, per liter of aqueous ferment.

2. The process of claim 1 characterized in that said yeast is selected from the genera Candida, Hansenula, Torulopsis, Saccharomyctes, Pichia, Debaryomyces, and Brettanomyces.

3. The process of claim 2 characterized in that said yeast is selected from the species Brettanomyces petrophilium, the Candida species C. boidinii, C. lipolytica, C. myoderma, C. utilis, C. stellatoidea, C. robusta, C. claussenii, C. rugosa and C. tropicalis, Debaryomyces hansenii, the Hansenula species H. minuta, H. saturnus, H. californica, H. mrakii, H. silvicola, H. polymorpha, H. wickerhamii, H. capsulata, H. glucozyma, H. henricii, H. nonfermentans and H. philodendra, the Pichia species P. farinosa, P. polymorpha, P. membranaefaciens, P. pinus, P. pastoris and P. trehalophilia, the Saccharomyces species S. cerevisiae, S. myces fragilis, S. rosei, S. acidifaciens, s. elegans, S. rouxill and Saccharomyces lactis, and the Torulopsis species. T. sonorensis, T. candida, T. bolmii, T. versatilis, T. opsis glabrata, T. molishiana, T. nemodendra, T. nitratophila and T. pinus.

4. The process of claim 2 characterized in that said yeast is selected from the genera Candida, Hansenula, Torulopsis, Pichia and Saccharomyces.

5. The process of claim 2 characterized by employing as said yeast a strain designated as or derived from one of Pichia pastoris NRRL Y-11430, Pichia pastoris NRRL Y-11431 and Hansenula polymorpha NRRL Y-11432.

6. The process of any of claims 1—5 characterized in that said carbohydrate is selected from at least one of glucose, fructose, galactose, lactose, sucrose, starch, and dextrin; said normal paraffin is selected from decane, undecane, dodecane, tridecane, tetradecane, pentadecane, hexadecane, octadecane, eicosane, or a mixture thereof; and said alcohol, ketone, aldehyde, acid, or ester is selected from methanol, ethanol, ethylene glycol, propylene glycol, 1-propanol, 2-propanol, glycerol 1-butanol, 2-butanol, 3-methyl-1-butanol, 1-pentanol, 2-hexanol, 1-7-heptanediol, 1-octanol, 2-decanol, 1-hexadecanol, 1-eicosanol, acetone 2-butanone, 4-methyl-2-pentanone, 2-decanone, 3-pentadecanone, 2-eicosanone, formaldehyde, acetaldehyde, propionaldehyde, butyraldehyde, hexanol, 7-methyloctanol, tetradecanal, eicosanal, acetic acid, propionic acid, butyric acid, glutaric acid, 5-methylhexanoic acid, azelaic acid, dodecanoic acid, eicosanoic acid, methyl formate, methyl acetate, ethyl acetate, propyl butyrate, isopropyl hexanoate, hexyl 5-methylloctanoate, octyl dodecanoate, or a mixture thereof.

7. The process of any of claims 1—5 characterized in that said carbon energy substrate is an alcohol of 1 to 4 carbon atoms.

8. The process of claim 7 characterized in that said alcohol is methanol or ethanol, preferably methanol.

9. The process of any of claims 1 to 8 characterized in that each liter of said ferment contains in the range of: P—1.9 to 20 g, K—1 to 20 g, Mg—0.15 to 3 g, Ca—0.06 to 1.6 g, S—0.1 to 8 g, Fe—6 to 140 mg, Zn—2 to 100 mg, Cu—0.6 to 16 mg, and Mn—0.6 to 20 mg.

10. The process of claim 9 characterized in that each liter of said ferment contains in the range of: P—2.2 to 10 g, K—1.5 to 10 g, Mg—0.3 to 1.2 g, Ca—0.08 to 0.8 g, S—0.2 to 5 g, Fe—9 to 80 mg, Zn—3 to 40 mg, Cu—1 to 10 mg, and Mn—0.9 to 8 mg.

11. The process of any of claims 1 to 10 characterized in that said aqueous ferment further contains at least one of sodium, cobalt, molybdenum, boron, and selenium.

12. The process of any of claims 1—11 characterized in that said aerobic fermentation conditions include the employment of any least one vitamin.

13. The process of claim 12 characterized in that said at least one vitamin is comprised of at least one of biotin and thiamine.

14. The process of claim 12 or 13 characterized in that said primary mineral salts medium contains compounds to supply nutrients comprising said P, K, Mg, S and Ca, and said trace mineral salts medium contains compounds to furnish nutrients comprising said Fe, Zn, Mn, and Cu, wherein said vitamin is admixed with an aqueous dilution of methanol or ethanol, the trace mineral medium is added thereto, and the resulting admixture is fed to the fermentation zone separated from said primary mineral salts medium.

15. The process of any of claims 1 to 14 characterized by employing a fermentation temperature in the range of 25 to 65°C, a pH in the range of 3 to 7, a pressure in the range of 0 to 1.03 MPa gauge, said fermentation conditions including a fermentation time in the range of 2 to 30 hours based on average retention.

16. The process of any of claims 1 to 15 characterized by maintaining a cell density of 70 to 150 grams, on a dried basis, per liter of fermentation admixture.

17. The process of any of claims 1 to 16 characterized by maintaining a cellular yield of 30 to 110 grams per 100 grams substrate charged.

18. The process of any of claims 1 to 17 characterized in that the fermentation effluent liquor including cells is subjected to a water-washing step, whereby the aqueous mineral medium remaining is substantially separated from the cells, leaving a wet cell mass, the wet cells are dried to produce dried cells, and recycling the water-washings and separated mineral medium to provide at least in part makeup water and mineral medium.

19. The process of any of claims 1—17 characterized in that the total fermentation effluent including cells and residual salts is dried, thereby producing a dried cell product containing residual water-soluble substances, including salts.

20. The process of any of claims 1—17 characterized by centrifuging the fermentation effluent liquor including cells, thereby producing a stream of concentrated cells, and a lean recycle liquor, water-washing said concentrated cell material, and drying said washed cells.

21. The process of claim 20 characterized in that at least one of the lean recycle liquor, or the water-washings from said water-washing step, at least in part is employed as at least a portion of said makeup medium.

22. A method of producing a single cell protein material which comprises culturing a yeast microrganism strain under aerobic fermentation conditions in an aqueous medium employing an oxygenated hydrocarbon as carbon energy source and recovering the resulting microorganisms as a single cell protein material characterized by employing as said yeast microorganism strain one of designated as or derived from Pichia pastoris NRRL Y-11430, Pichia pastoris NRRL Y-11431 and Hansenula polymorpha NRRL Y-11432.

23. A culture obtained by propagation of and having the identifying characteristics of Pichia pastoris NRRLY-11430.

24. A culture obtained by propagation of and having the identifying characteristics of Pichia pastoris NRRL Y-11431.

25. A culture obtained by propagation of and having the identifying characteristics of Hansenula polymorpha NRRL Y-11432.

## Patentansprüche

1. Kontinuierliches Verfahren zur Herstellung von Single Cell Protein (SCP) durch Kultivieren unter aeroben Fermentationsbedingungen in wässrigem Mineralsalz-Fermentationsmedium mindestens einer Hefespecies unter Anwendung von für Hefe wirksamen Kultivierungs-Fermentationsbedingungen in Gegenwart eines Kohlenstoff-Energiesubstrats, einer assimilierbaren Stickstoffquelle, Wasser, Sauerstoff und eines wässrigen Mineralsalzmediums, das ein primäres Mineralsalzmedium und ein Spurenmineralsalzmedium umfaßt, sowie kontinuierliches Austragen von wäßrigem Fermentationsmedium zum Sammeln der erhaltenen Mikroorganismen als SCP, wobei das Kohlenstoff-Energiesubstrat ausgewählt ist aus Kohlenhydraten, Alkoholen, Ketonen, Aldehyden, Säuren und Estern mit 1 bis 20 C-Atomen pro Molékul, und n-Paraffinen von 10 bis 20 C-Atomen pro Molekül, dadurch gekennzeichnet, daß man die Mineralsalze dem wässrigen Fermentationsmedium in solcher Menge zuführt, daß in dem wässrigen Fermentationsmedium folgende Elemente in mindestens folgenden Mengen, jeweils pro Liter wässriges Fermentationsmedium vorliegen: P—1,9 g, K—1 g, Mg—0,15 g, Ca—0.06 g, S—0,1 g, Fe— 6 mg, Zn—2 mg, Cu—0,6 mg, und Mn 0,6 mg, wodurch eine Zelldichte in dem wässrigen Fermentationsmedium von mindestens 60 und bis zu 160 g pro Liter wässriges Fermentationsmedium, bezogen auf Trockensubstanz, aufrechterhalten wird.

2. Verfahren nach Anspruch 1, dadurch gekennzeichnet, daß die Hefe ausgewählt ist aus den Gattungen Candida, Hansenula, Torulopsis, Saccharomyces, Pichia, Debaryomyces und Brettanomyces.

3. Verfahren nach Anspruch 2, dadurch gekennzeichnet, daß die Hefe ausgewählt ist aus den Spezies Brettanomyces petrophilium, den Candida-Spezies C. boidinii, C. lipolytica, C. myoderma, C. utilis, C. stellatoidea, C. robusta, C. claussenii, C. rugosa und C. tropicalis, Debaryomyces hansenii, den Hansenula-Spezies H. minuta, H. saturnus, H. californica, H. markii, H. silvicola, H. polymorpha, H. wickerhamii, H. capsulata, H. glucozyma, H. henricii, H. nonfermentans und H. philodendra, den Pichia-Spezies P. farinosa, P. polymorpha, P. membranaefaciens, P. pinus, P. pastoris und P. trehalophila, den Saccharomyces-Spezies S. cerevisiae, S. myces fragilis, S. rosei, S. acidifaciens, S. elegans, S. rouxii und S. lactis, und den Torulopsis-Spezies T. sonorensis, T. candida, T. bolmii, T. versatilis. T. opsis glabrata, T. molishiana, T. nemodendra, T. nitratophila und T. pinus.

4. Verfahren nach Anspruch 2, dadurch gekennzeichnet, daß die Hefe ausfewählt ist aus den Gattungen Candida, Hansenula, Torulopsis, Pichia und Saccharomyces.

5. Verfahren nach Anspruch 2, dadurch gekennzeichnet, daß man als Hefe einen Stamm verwendet, der bezeichnet ist als oder erhalten worden ist von Pichia pastoris NRRL Y-11430, Pichia pastoris NRRL Y-11431 oder Hansenula polymorpha NRRL Y-11432.

6. Verfahren nach einem der Ansprüche 1 bis 5, dadurch gekennzeichnet, daß das Kohlenhydrat ausgewählt ist aus mindestens einem von Glucose, Fructose, Galactose, Lactose, Saccharose, Stärke und Dextrin; das n-Paraffin ausgewählt ist aus Decan, Undecan, Dodecan, Tridecan, Tetradecan, Pentadecan, Hexadecan, Octadecan, Eicosan oder einem Gemisch hiervon; und Alkohol, Keton, Aldehyd, Säure oder Ester ausgewählt sind aus Methanol, Ethanol, Ethylenglycol, Propylenglycol, 1-Propanol, 2-Propanol, Glycerin, 1-Butanol, 2-Butanol, 3-Methyl-1-butanol, 1-Pentanol, 2-Hexanol, 1-7-Heptandiol, 1-Octanol, 2-Decanol, 1-Hexadecanol, 1-Eicosanol, Aceton, 2-Butanon, 4-Methyl-2-pentanon, 2-decanon, 3-Pentadecanon, 2-Eicosanon, Formaldehyd, Acetaldehyd, Propionaldehyd, Butryraldehyde, Hexanol 7-Methyloctanol, Tetradecanal, Eicosanal, Essigsäure, Propionsäure, Buttersäure, Glutarsäure, 6-Methylhexansäure, Azelainsäure, Dodecansäure, Eicosansäure, Methylformiat, Methylacetat, Ethylacetat, Propylbutyrat, isopropylhexanoat, Hexyl-5-methyl-octanoat, Octyldodecanoat, oder einem Gemisch hiervon.

7. Verfahren nach einem der Ansprüche 1 bis 5, dadurch gekennzeichnet, daß das Kohlenstoff-Energiesubstrat ein Alkohol mit 1 bis 4 C-Atomen ist.

8. Verfahren nach Anspruch 7, dadurch gekennzeichnet, daß der Alkohol Methanol oder Ethanol, vorzugsweise Methanol, ist.

9. Verfahren nach einem der Ansprüche 1 bis 8, dadurch gekennzeichnet, daß pro Liter Fermentationsmedium enthalten sind; P, 1,9—20 g, K 1—20 g, Mg, 0,15—3 g, Ca 0,06—1.6 g, S 0,1—8 g, Fe 6—140 mg, Zn 2—100 mg, Cu 0,6—16 mg, Mn 0,6—20 mg.

10. Verfahren nach Anspruch 9, dadurch gekennzeichnet, daß pro Liter Fermentationsmedium enthalten sind; P 2,2—10 g K 1,5—10 g., Mg 0,3—1,2 g Ca 0,08—0,8 g S, 0,2—5 g, Fe 9—80 mg, Zn 3—40 mg, Cu 1—10 mg, und Mn 0.9—8 mg.

11. Verfahren nach einem der Ansprüche 1 bis 10, dadurch gekennzeichnet, daß das wässrige Fermentationsmedium weiterhin mindestens einen Bestandteil aus der Gruppe Natrium, Kobalt, Molybdän, Bor und Selen enthält.

12. Verfahren nach einem der Ansprüche 1 bis 11, dadurch gekennzeichnet, daß die aeroben Fermentationsbedingungen die Verwendung mindestens eines Vitamins einschließen.

13. Verfahren nach Anspruch 12, dadurch gekennzeichnet, daß als Vitamine mindestens eines von Biotin und Thiamin anwesend sind.

14. Verfahren nach Anspruch 12 oder 13, dadurch gekennzeichnet, daß das primäre Mineralsalzmedium Verbindungen enthält, die Nährstoffe mit P, K, Kg, S und Ca liefern, und das Spurenmineralsalzmedium Verbindungen enthält, die Nährstoffe mit Fe, Zn, Mn und Cu liefern, wobei das Vitamin vermischt ist mit wässrig verdünntem Methanol oder Ethanol hierzu das Spurenmineralsalzmedium zugesetzt wird, und das erhaltene Gemisch der Fermentationszone getrennt von dem primären Mineralsalzmedium zugeführt wird.

15. Verfahren nach einem der Ansprüche 1 bis 14, dadurch gekennzeichnet, daß man eine Fermentationstemperatur im Bereich von 25 bis 65°C, einen pH-Wert im Bereich von 3 bis 7 einen Druck im Bereich von 0 bis 1,03 MPa (Überdruck) und Fermentationszeiten von 2 bis 30 Stunden, bezogen auf mittlere Verweilzeit, anwendet.

16. Verfahren nach einem der Ansprüche 1 bis 15, dadurch gekennzeichnet, daß man eine Zelldichte von 70 bis 150 g, bezogen auf Trockensubstanz, pro Liter Fermentationsmedium aufrechterhält.

17. Verfahren nach einem der Ansprüche 1 bis 16, dadurch gekennzeichnet, daß man eine Zellausbeute von 30 bis 110 g pro 100 g eingesetztes Substrat aufrechterhält.

18. Verfahren nach einem der Ansprüche 1 bis 17, dadurch gekennzeichnet, daß die ausgetragene zellhaltige Fermentationsbrühe eine Wasserwäsche unterworfen wird, wobei das verbliebene wässrige Mineralsalzmedium im wesentlichen von den Zellen unter Erhalt einer nassen Zellmasse abgetrennt wird, die nassen Zellen einer Trocknung unter Erhalt von getrockneten Zellen unterworfen werden, und die Waschwässer und das abgetrennte Mineralsalzmedium in den Prozeß zurückgespeist werden und so zumindest teilweise Zubereitungswasser und Mineralsalzmedium liefern.

19. Verfahren nach einem der Ansprüche 1 bis 17 dadurch gekennzeichnet, daß der gesamte Fermentationsaustrag einschließlich Zellen und restliche Salze getrocknet wird, wobei man ein getrocknetes Zellprodukt erhält das restliche wasserlösliche Stoffe, einschließlich Salze, enthält.

20. Verfahren nach einem der Ansprüche 1 bis 17, dadurch gekennzeichnet, daß man die ausgetragene Fermentationsbrühe einschließlich Zellen zentrifugiert, wobei ein Strom von konzentrierten Zellen und eine verdünnte Rückspeiseflüssigkeit entstehen, das konzentrierte Zellmaterial der Wasserwäsche unterwirft, und die gewaschenen Zellen trocknet.

21. Verfahren nach Anspruch 20, dadurch gekennzeichnet, daß man mindestens einen der Ströme verdünnte Rückspeiseflüssigkeit und Waschwässer aus der Wasser-Waschstufe zumindest teilweise als zumindest einen Teil der Ergänzungsmedium verwendet.

22. Verfahren zur Herstellung von Single Cell Protein (SCP) durch Kultivieren eines Hefestammes unter aeroben Fermentationsbedingungen in wässrigem Medium unter Verwendung eines oxidierten Kohlenwasserstoffs als Kohlenstoff-Energiequelle und Sammeln der erhaltenen Mikroorganismen als SCP, dadurch gekennzeichnet, daß man einen Hefestamm verwendet, der bezeichnet ist als oder erhalten worden ist von Pichia pastoris NRRL Y-11430, Pichia pastoris NRRL Y-11431 oder Hansenula polymorpha NRRL Y-11432.

23. Kultur, erhalten durch Vermehrung von und mit den kennzeichnenden Eigenschaften von Pichia pastoris NRRL Y-11430.

24. Kultur, erhalten durch Vermehrung von und mit den kennzeichnenden Eigenschaften von Pichia pastoris NRRL Y-11431.

25. Kultur, erhalten durch Vermehrung von und mit den kennzeichnenden Eigenschaften von Hansenula polymorpha NRRL Y-11432.

**Revendications**

1. Procédé continu pour produire une matière protéinée unicellulaire qui consiste à cultiver dans des conditions de fermentation aérobie dans un ferment aqueux de sels minéraux au moins une espèce de levure en employant des conditions efficaces de fermentation pour la culture de levure, comprenant des quantités de substrat énergétique carboné, de source d'azote assimilable, d'eau d'appoint, d'oxygène et de milieu aqueux de sels minéraux comprenant un milieu principal de sels minéraux et un milieu de sels minéraux à l'état de trace, et à retirer de façon continue du ferment aqueux pour récupérer les microorganismes résultants en tant que matière protéinée unicellulaire, où le substrat énergétique carboné est choisi parmi des hydrates de carbone, des alcools, des cétones, des aldéhydes, des acides et des esters ayant 1 à 20 atomes de carbone par molécule, et des paraffines normales ayant 10 à 20 atomes de carbone par molécule, caractérisé en ce qu'on envoie les sels minéraux au ferment aqueux à un taux tel que l'on maintient dans le ferment aqueux les éléments suivants au moins suivant les poids désignés par litre de ferment aqueux: P—1,9 g, K—1 g, Mg—0,15 g, Ca—0,06 g, S—0,1 g, Fe—6 mg, Zn—2 mg, Cu—0,6 mg et Mn—0,6 mg en maintenant ainsi une densité de cellules dans le ferment aqueux d'au moins 60 et jusqu'à 160 grammes, sur une base séchée, par litre de ferment aqueux.

2. Procédé selon la revendication 1, caractérisé en ce que la levure est choisie parmi les genres Candida, Hansenula, Torulopsis, Saccharomyces, Pichia, Debaryomyces et Brettanomyces.

3. Procédé selon la revendication 2, caractérisé en ce que la levure est choisie parmi l'espéce Brettanomyces petrophilium, l'espèce Candida C. Boidinii, C. lipolytica C. myoderma, C. utilis, C. Stellatoidea, C. robusta, C. claussenii, C. rugosa et C. tropicalis Debaryomyces hansenii, l'espèce Hansenula H. minuta, H. saturnus, H. californica, H. mrakii, H. silvicola, H. polymorpha, H. wickerhamii, H. capsulata, H. glucozyma, H. henricii, H. nonfermentans et H. philodendra, l'espèce Pichia P. farinosa, P. polymorpha, P. membranaefaciens, P. pinus, P pastoris et P. trehalophila, l'espèce Saccharomyces S. cerevisiae, S. myces fragilis, S. rosei, S. acidifaciens, S. elegans, S. rouxii et Saccharomyces lactis, et l'espèce Torulopsis T. sonorensis T. candida, T. bolmii, T. versatilis, T. opsis glabrata, T molishiana. T. nemodendra, T. nitratophila et T. pinus.

4. Procédé selon la revendication 2, caractérisé en ce que la levure est choisie parmi les genres Candida, Hansenula, Torulopsis, Pichia et Saccharomyces.

5. Procédé selon la revendication 2, caractérisé en ce qu'on emploie comme levure une souche désignée par, ou dérivée d'une souche de Pichia pastoris NRRI Y-11430, Pichia pastoris NRRL Y-11431 et Hansenula polymorpha NRRL Y-11432.

6. Procédé selon l'une quelconque des revendications 1 à 5, caractérisé en ce que l'hydrate de carbone est choisi parmi au moins un des produits formés par le glucose, le fructose, le galactose, le lactose, le saccharose, l'amidon et le dextrine; la paraffine normale est choisie parmi le décane, l'undécane, le dodécane, le tridécane, le tétradécane, le pentadécane l'hexadécane, l'octadécane, l'éicosane, ou leur mélange; et l'alcool, le cétone, l'aldéhyde, l'acide ou l'ester est choisi parmi le méthanol, léthanol, l'éthylèneglycol, le propylèneglycol, le 1-propanol, le 2-propanol, le glycérol, le 1-butanol, le 2-butanol, le 3-méthyl-1-butanol, le 1-pentanol, le 2-hexanol, le 1,7-heptanediol, le 1-octanol, le 2-décanol, le 1-hexadécanol, le 1-éicosanol, l'acétone, la 2-butanone, la 4-méthyl-2-pentanone, la 2-décanone, la 3-pentadécanone, la 2-éiocasanone, le formaldéhyde l'acétaldéhyde, le propionaldéhyde, le butyraldéhyde, l'hexanol, le 7-méthyloctanol, le tétradécanal, l'éicosanal, l'acide acétique, l'acide propionique, l'acide butyrique, l'acide glutarique, l'acide 5-méthylhexanoîque, l'acide azélaîque, l'acide dodécanoîque, l'acide éicosanoîque, le formiate de méthyle, l'acétate de méthyle, l'acétate d'éthyle, le butyrate, de propyle, l'hexanoate d'isopropyle, le 5-méthyloctanoate d'hexyle, le dodécanoate d'octyle ou leur mélange.

7. Procédé selon l'une quelconque des revendications 1 à 5, caractérisé en ce que le substrat énergétique carboné est un alcool ayant 1 à 4 atomes de carbone.

8. Procédé selon la revendication 7, caractérisé en ce que l'alcool est le méthanol ou l'éthanol, de préférence le méthanol.

9. Procédé selon l'une quelconque des revendications 1 à 8, caractérisé en ce que chaque litre de ferment contient dans la gamme de: P—1,9 à 20 g K—1 à 20 g, Mg—0,15 à 3 g, Ca—0,06 à 1,6 g, S—0,1 à 8 g, Fe—6 à 140 mg, Zn—2 à 100 mg, Cu—0,6 à 16 mg, et Mn—0,6 à 20 mg.

10. Procédé selon la revendication 9, caractérisé en ce que chaque litre de ferment contient dans la gamme de: P—2,2 à 10 g, K—1,5 à 10 g, Mg—0,3 à 1,2 g, Ca—0,08 à 0,8 g, S—0,2 à 5 g, Fe—9 à 80 mg, Zn—3 à 40 mg, Cu—1 à 10 mg, et Mn—0,9 à 8 mg.

11. Procédé selon l'une quelconque des revendications 1 à 10, caractérisé en ce que le ferment aqueux contient en outre au moins un des éléments sodium, cobalt, molybdéne, bore et sélénium.

12. Procédé selon l'une quelconque des revendications 1 à 11, caractérisé en ce que les conditions de fermentation aérobies comprennent l'emploi d'au moins une vitamine.

13. Procédé selon la revendication 12, caractérisé en ce qu'au moins une vitamine est constituée d'au moins un des produits formés par la biotine et la thiamine.

14. Procédé selon la revendication 12 ou la revendication 12, caractérisé en ce que le milieu principal de sels minéraux contient des composés pour fournir des produits nutritifs comprenant P, K, Mg, S et Ca, et le milieu de produits minéraux à l'état de trace contient des composés pour fournir des produits nutritifs comprenant Fe, Zn, Mn et Cu, où la vitamine est mélangée avec une dilution aqueuse de méthanol ou

d'éthanol, le milieu de produits minéraux à l'état de trace y est ajouté, et le mélange résultant est envoyé à la zone de fermentation séparée du milieu principal de sels minéraux.

15. Procédé selon l'une quelconque des revendications 1 à 14, caractérisé en ce qu'on emploie une température de fermentation dans l'intervalle de 25 à 65°C, un pH dans la gamme de 3 à 7, une pression dans la gamme de 0 à 1,03 MPa au-dessus de la pression atmosphérique, les conditions de fermentation comprenant un temps de fermentation dans la gamme de 2 à 30 heures, en se basant sur la rétention moyenne.

16. Procédé selon l'une quelconque des revendications 1 à 15, caractérisé en ce qu'on maintient une densité de cellules de 70 à 150 grammes, sur une base séchée, par litre du mélange de fermentation.

17. Procédé selon l'une quelconque des revendications 1 à 16, caractérisé en ce qu'on maintient un rendement de cellules de 30 à 110 grammes pour 100 grammes de substrat chargé.

18. Procédé selon l'une quelconque des revendications 1 à 17, caractérisé en ce que la liqueur d'effluent de fermentation comprenant les cellules est soumise à une étape de lavage par l'eau, et, de ce fait, le milieu aqueux de produits minéraux restant est sensiblement séparé des cellules, en laissant une masse de cellules humides, le cellules humides sont séchées pour produire des cellules séchées, en on recycle les lavages aqueux et le milieu de produits minéraux séparé pour fournir au moins en partie l'eau d'appoint et le milieu de produits minéraux.

19. Procédé selon l'une quelconque des revendications 1 à 17, caractérisé en ce que l'effluent de fermentation total comprenant les cellules et les sels résiduels est séché, en produisant ainsi un produit de cellules séchées contenant de substances résiduelles solubles dans l'eau, y compris de sels.

20. Procédé selon l'une quelconque des revendications 1 à 17, caractérisé en ce qu'on centrifuge la liqueur d'effluent de fermentation comprenant des cellules, en produisant ainsi un courant de cellules concentrées, et une liqueur de recyclage diluée, on lave par l'eau la matière de cellules concentrées et on sèche le cullules lavées.

21. Procédé selon la revendication 20, caractérisé en ce qu'au moins un des produits formés par la liqueur de recyclage diluée, ou les lavages par l'eau provenant de l'étape de lavage par l'eau au moins en partie est employé comme au moins une partie du milieu d'appoint.

22. Procédé pour cultiver une matière protéinée unicellulaire qui consiste à cultiver une souche de microorganisme de levure dans des conditions de fermentation aérobie dans un milieu aqueux en employant un hydrocarbure oxygéné comme source énergétique carbonée et à récupérer les microorganismes résultants sous forme d'une matière protéinée unicellulaire, caractérisé en ce qu'on emploie comme souche de microorganismes de levure, une levure désignée par ou dérivée de Pichia pastoris NRRL Y-11430, de Pichia pastoris NRRL Y-11431 et d'Hansenula polymorpha NRRL Y-11432.

23. Culture obtenue par propagation de et ayant les caracteristiques d'identification du Pichia pastoris NRRL Y-11430.

24. Culture obtenue par propagation de et ayant les caractéristiques d'identification du Pichia pastoris NRRL Y-11431.

25. Culture obtenue par propagation de et ayant les caractéristiques d'identification de l'Hansenula polymorpha NRRL Y-11432.